Europäisches Patentamt

European Patent Office

Office européen des brevets

(19)

(11) **EP 1 074 279 A1**

(12) **EUROPEAN PATENT APPLICATION**
published in accordance with Art. 158(3) EPC

(43) Date of publication:
**07.02.2001 Bulletin 2001/06**

(51) Int. Cl.⁷: **A63B 22/06**, A63B 23/04,
A63B 71/06, A61B 5/05

(21) Application number: **99917188.7**

(22) Date of filing: **27.04.1999**

(86) International application number:
**PCT/JP99/02247**

(87) International publication number:
**WO 99/55429 (04.11.1999 Gazette 1999/44)**

(84) Designated Contracting States:
**AT BE CH CY DE DK ES FI FR GB GR IE IT LI LU MC NL PT SE**

(30) Priority: **28.04.1998 JP 11973498**

(71) Applicant: **Yaman Ltd.**
**Tokyo 135-0045 (JP)**

(72) Inventors:
• **YAMAZAKI, Iwao**
**Koto-ku, Tokyo 135-0045 (JP)**

• **YAMAZAKI, Kimiyo**
**Koto-ku, Tokyo 135-0045 (JP)**

(74) Representative:
**Newstead, Michael John et al**
**Page Hargrave**
**Southgate**
**Whitefriars**
**Lewins Mead**
**Bristol BS1 2NT (GB)**

(54) **EXERCISE EFFECT DISPLAY DEVICE FOR AEROBIC EXERCISE MACHINE**

(57) Disclosed is an exercise effect display for an aerobic exercise machine intended to allow one to use any part of one's body for strengthening one's body.

It comprises: bodily impedance measuring means 11 having grip-shaped electrodes H to be applied to selected parts of one's body for determining the bodily impedance; data inputting means 12 for inputting individual data pertaining to sex, age, height and weight with the aid of a colour liquid crystal panel C; means 13 for determining a bodily fat rate from the bodily impedance and the individual data; memory means 14 for storing the bodily impedance for each measurement; and display means 15 for providing a time-sequential display of so stored bodily impedances.

With this arrangement one can realize the bodily effect caused by taking a bodily exercise for a long time of period.

FIG. 5

EP 1 074 279 A1

## Description

### Technical Field

The present invention relates to aerobic exercise machines intended to strengthen the heart and lung for improving one's physical endurance, and at the same time intended to cause extra amount of bodily fat to be burnt by supplying the physical body with as much oxygen as can be supplied by using any part of one's body. Such exercise machines include a treadmill for walking or jogging, a bicycle ergometer for pedalling or a stair climber for raising one's weight.

### Background Art

What is aimed at by taking an aerobic exercise is to waste extra quantity of bodily fat for giving a nice shape in addition to the strengthening of the heart and lung for improving the physical endurance

Assume that an aerobic exercise is taken for reducing one's bodily weight, and that such aerobic exercise has been continued a relatively long time of period. Then, if the exercise effect can be realized particularly in terms of reduction of bodily weight, the person taking such aerobic exercise will be encouraged.

Conventional aerobic exercise machines are equipped with displays for showing instantaneous data pertaining to the degree of loading, the amount of exercise and other exercise or physical conditions, but no exercise machines are equipped with any display for showing the effect of exercise caused by continuing a bodily exercise for a relatively long time of period for instance, in the hope of improving physical beauty. Therefore, persons taking such aerobic exercise are apt to give up it; they are discouraged because no goal is unseen.

In view of the above one object of the present invention is to provide a display which is capable of showing a time-sequential change of physical effect caused by the long-termed continuance of aerobic exercise, such physical effect being estimated in terms of reduction of bodily fat.

### Disclosure of the Invention

To attain this object an exercise effect display for an aerobic exercise machine intended to allow one to use any part of one's body for strengthening one's body comprises: bodily impedance measuring means having electrodes to be applied to selected parts of one's body for determining the bodily impedance; data inputting means for inputting individual data pertaining to sex, age, height and weight; means for determining a bodily fat rate from the bodily impedance and the individual data; memory means for storing the bodily impedance for each measurement; and display means for providing a time-sequential display of so stored bodily impedances.

### Brief Description of Drawings

Fig. 1 shows diagrammatically a treadmill for walking or jogging;
Fig.2 shows diagrammatically a bicycle ergometer for pedalling;
Fig.3 shows diagrammatically a stair climber for raising one's weight;

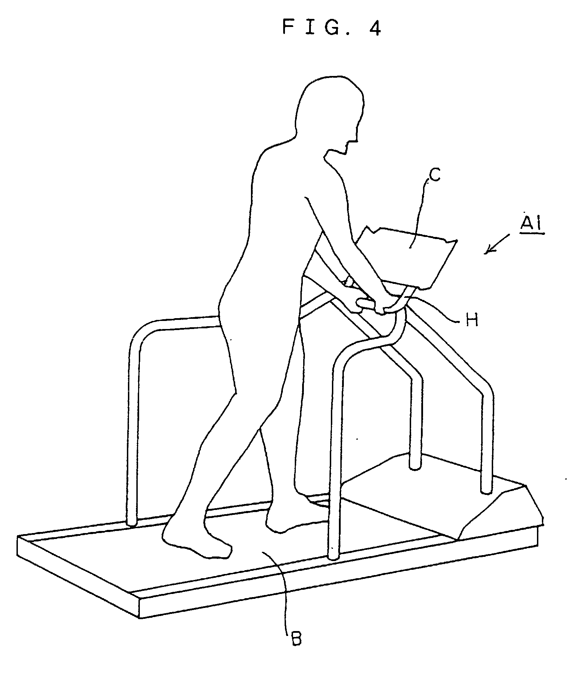

Fig.4 shows diagrammatically a treadmill, the crossbar of which is held with both hands of the person using the machine for required measurement;
Fig.5 shows the structure of the aerobic exercise effect display;
Fig.6 is an enlarged view of the crossbar;
Fig.7 shows a bodily impedance measuring circuit;
Fig.8 shows how a time-sequential display of bodily fat is given on the display; and
Fig.9 shows how a time-sequential display of bodily weight is given on the display.

### Best Mode of Reducing the Invention into Practice

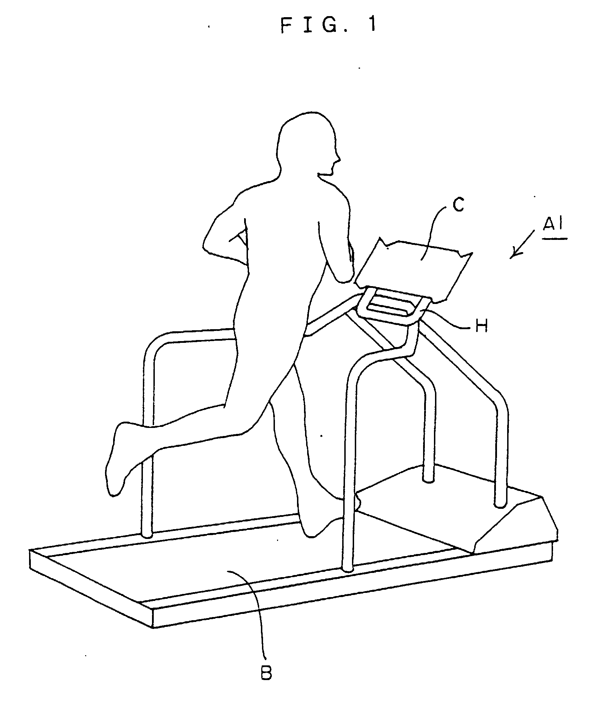

Figs.1 to 3 illustrate different aerobic exercise machines to which the present invention can be applied.

Referring to Fig.1, a person walks or runs on the running belt B of the treadmill A1.

The treadmill A1 has a colour liquid crystal panel C mounted on its front side. The colour liquid crystal display C is responsive to finger touch for showing the loading condition, the speed or gradient of the running belt B, the heartbeat rate and other exercise or physical data. The person can be loaded appropriately by controlling the gradient or speed of the running belt B. Measurements of bodily impedance and heartbeat rate can be effected by holding the crossbar H with hands.

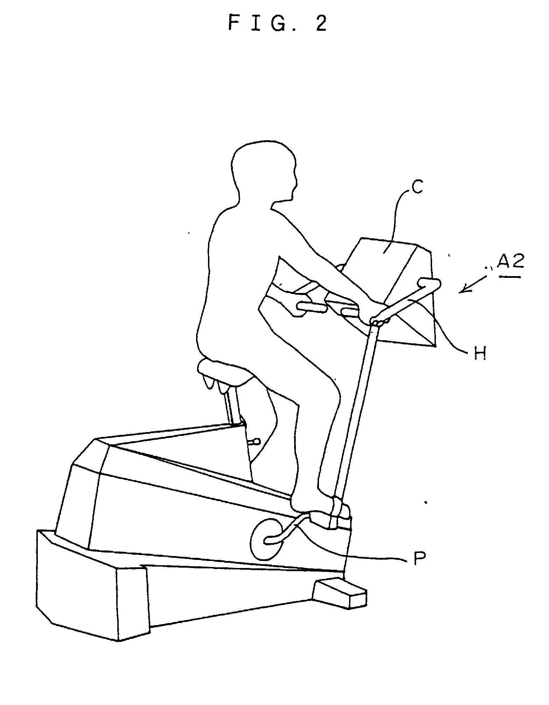

Referring to Fig.2, the person rides the bicycle ergometer A2, turning the pedals P on the bicycle.

The bicycle ergometer A2 has a colour liquid crystal panel C on its front side. It shows the loading condition, the speed or gradient of the running belt B, the heartbeat rate and other exercise or physical data. The person can be loaded appropriately by controlling the friction resistance given by the pedals P. Measurements of bodily impedance and heartbeat rate can be effected by holding the crossbar H with hands.

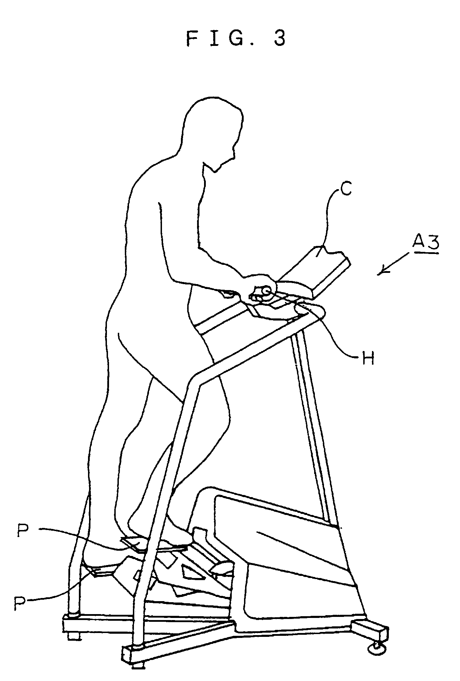

Referring to Fig.3, the person pushes down two adjacent pedals P alternately with feet. The stair climber A3 has a colour liquid crystal panel C on its front side. It shows the loading condition, the speed or gradient of the running belt B, the heartbeat rate and other exercise or physical data. The person can be loaded appropriately by controlling the weight of the pedals P. Measurements of bodily impedance and heartbeat rate can be effected by holding the crossbar H with hands.

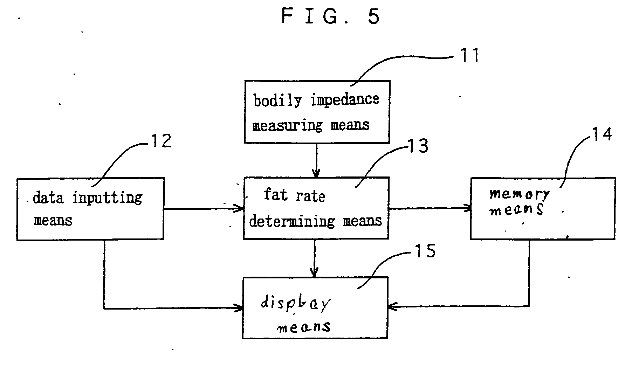

Referring to Fig.5, an aerobic exercise effect display 1 according to the present invention comprises bodily impedance measuring means 11 for determining a bodily impedance with the aid of the electrodes of the crossbar H, data input means 12 for inputting individual data pertaining to sex, age, height and weight with the aid of the colour liquid crystal display C, bodily fat rate calculating means 13 for determining a bodily fat rate both from the bodily impedance and the individual data, memory means 14 for storing the bodily fat rate for every measurement, and display means 15 for providing a time-sequential graph of bodily fat rate.

While taking a bodily exercise one's weight can be determined by using a strain gauge, which may be installed in the exercise machine.

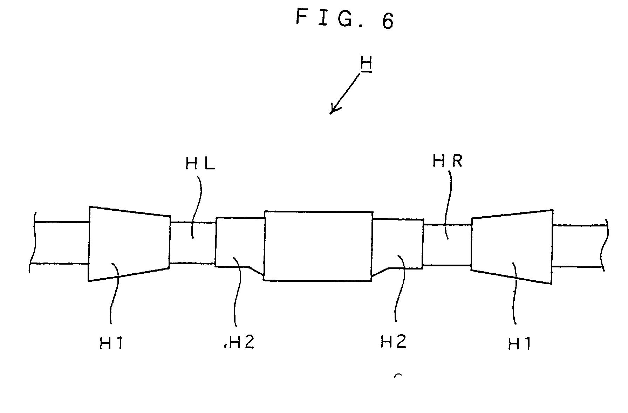

Referring to Fig.6, the grip crossbar H comprises an insulating rod having left and right grips HL and HR formed on its opposite sides, and these grips HL and HR have feeding electrodes H1 and H1 and detecting electrodes H2 and H2 applied therearound. The feeding electrodes H1 and H1 are separated from the counter detecting electrodes H2 and H2.

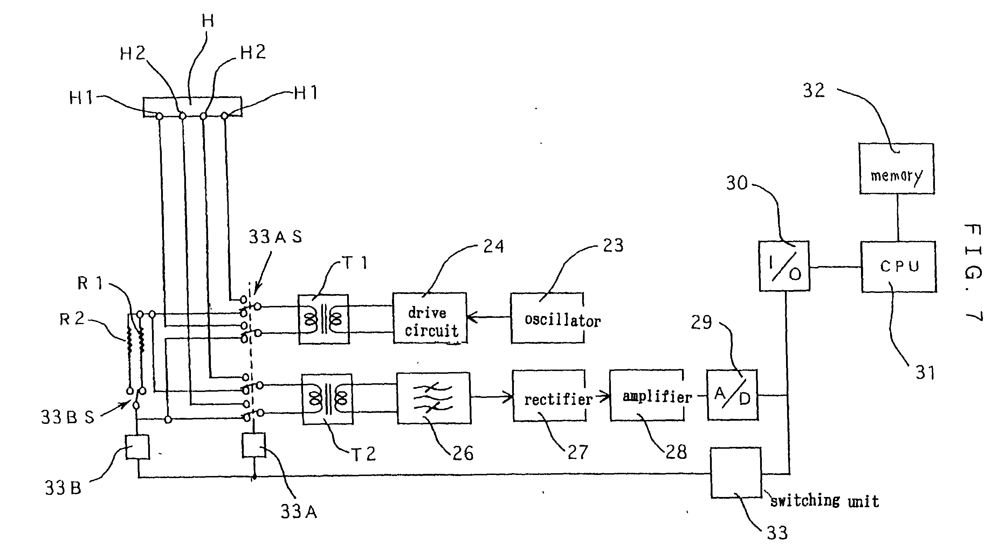

Fig.7 is a block diagram of a measuring circuit of the bodily impedance measuring means 11. As shown in the drawing, an oscillator 21 generates sinusoidal voltage at 50 kHz, which is fed to the feeding electrodes H1 and H1 via a drive circuit 22, a transformer T1 and a switch 23A. When a person holds the left and right feeding electrodes H1 and H1 in his hands, an ac voltage appears between the detecting electrodes H2 and H2. The ac voltage is converted to a corresponding dc voltage after passing through the switch 23A, a transformer T2, a band filter 24, a rectifier 25 and an amplifier 26, and then, the dc voltage is directed to a CPU 4 via an A/D converter 27 and an I/O interface 6 after being shaped, level-controlled and offset-adjusted. The CPU 4 has a memory 5 associated therewith.

In an attempt to reduce a measurement error which would be caused by the time variability and temperature characteristics of some circuit components the measuring circuit must be calibrated in terms of its input-to-output characteristics prior to measurement of bodily impedance. Specifically the calibration can be effected by using the following equation:

$$Z = kV + C_0$$

where Z stands for bodily impedance; V stands for the voltage appearing between the detecting electrodes H2 and H2; k stands for a proportionality factor; and $C_0$ stands for a fixed constant.

The same ac voltage as used in measuring bodily impedance Z is applied across two resistances R1 and R2 of known values alternately to determine the voltages appearing across these known resistances R1 and R2. By substituting the known values of R1 and R2 and the so determined voltages for Z and V in two equations two contacts k and $C_0$ can be determined.

To do this the CPU 4 directs a control signal to the switching control 29A via the I/O interface 6 and a switching unit 28 to put the resistance R1 in the measuring circuit. Then, the CPU 4 directs another control signal to the switching control 29R via the I/O interface 6 and the switching unit 28 to put the resistance R2 in the measurement circuit.

The bodily impedance measuring means 11 can determine a bodily impedance according to the following equation 1:

Bodily Impedance = A x Gradient of the Curve x Measured Value + Ordinates Intersection of the Curve

| A = 0.792 | male |
|---|---|
| 0.825 | female |

$$\text{Gradient} = (810\text{-}310)/(\text{Upper Measured Value} - \text{Lower Measured Value})$$

$$\text{Intersection} = 810 - \text{Gradient} \times \text{Upper Measured Value}$$

The bodily fat rate measuring means 13 can determine a bodily fat rate according to the following equation 2:

Females:

$$\text{Bodily Fat Rate} = (1 - \text{Non-Fat Texture/Weight}) \times 100$$

$$\text{Non-Fat Texture} = 0.6483 \times (\text{Height} \times \text{Height})/\text{Impedance} + B \times \text{Weight} + 5.091$$

| B = 0.1699 | for Height: 150cm or taller | |
|---|---|---|
| 0.12 | Height: below 150cm | Upper Limit of Ideal Weight + 2.5 or more |
| 0.13 | | Upper Limit of Ideal Weight + 2.0 or more |
| 0.14 | | Upper Limit of Ideal Weight + 1.5 or more |
| 0.15 | | Upper Limit of Ideal Weight + 1.0 or more |
| 0.16 | | above Upper Limit of Ideal Weight |
| 0.1699 | | below Upper Limit of Ideal Weight |

$$\text{Upper Limit of Ideal Weight} = (\text{Height} - 100) \times 0.9 \times 1.1$$

Males:

$$\text{Bodily Fat Rate} = (4.95/\text{Bodily Density} - 4.5) \times 100$$

$$\text{Bodily Density} = 1.1554 - (0.0841 \times \text{Weight} \times \text{Impedance})/(\text{Weight} \times \text{Height})$$

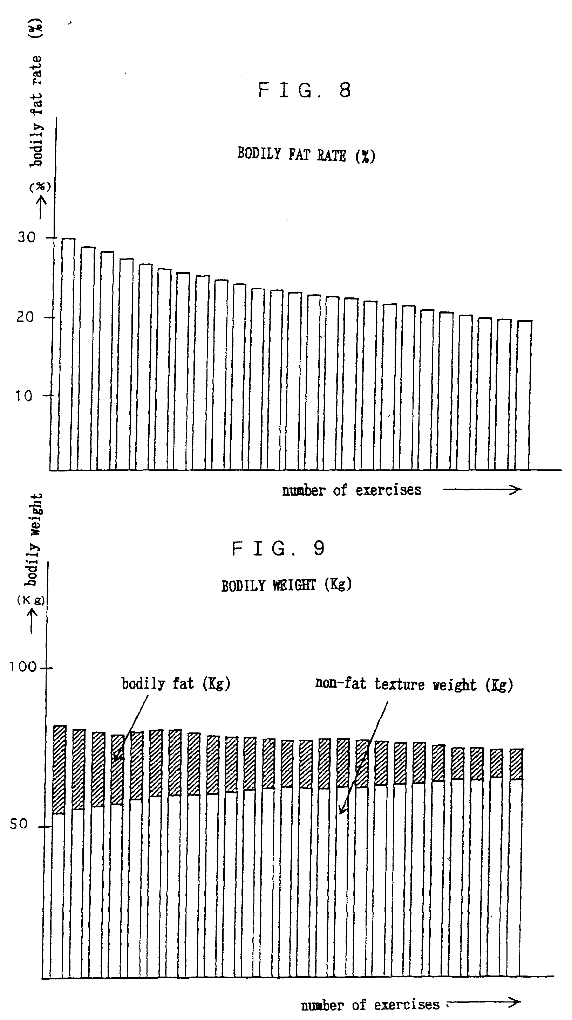

Referring to Fig.8, the display means 15 shows a time-sequential graph of bodily fat rate on the colour liquid crystal panel C. The bar graph has the bodily fat rate (%) and the number of exercises on its ordinates and abscissa respectively.

Referring to Fig.9, the display means 15 shows a time-sequential graph of bodily weight on the colour liquid crystal panel C. The bar graph has the bodily weight (kg) and the number of exercises on its ordinates and abscissa respectively. The bodily weight is composed of bodily fat weight (kg) and non-fat texture weight (kg).

**Possibility of Industrial Utility**

As may be understood from the above, an aerobic exercise effect display according to the present invention can determine a bodily impedance from individual data pertaining to sex, age, height and weight to calculate a bodily fat rate from the so determined bodily impedance. The bodily fat rates are stored to provide a time-sequential graph of bodily fat rates on the display.

Thus, a person taking an aerobic exercise can realize the effect of continuing such an aerobic exercise in terms of how his bodily fat has varied, so that the person may be encouraged at the result thus attained. Thus, the person can continue the exercise eagerly until he has attained his purpose.

Also advantageously, he can visually realize how his bodily fat has been reduced, and he is increasingly eager to take such an exercise. As a result his bodily fat can be reduced significantly in a short length of time.

**Claims**

1. Exercise effect display for an aerobic exercise machine intended to allow one to use any part of one's body for strengthening one's body comprising:

   bodily impedance measuring means having electrodes to be applied to selected parts of one's body for determining the bodily impedance;
   data inputting means for inputting individual data pertaining to sex, age, height and weight;
   means for determining a bodily fat rate from the bodily impedance and the individual data;
   memory means for storing the bodily impedance for each measurement; and
   display means for providing a time-sequential display of so stored bodily impedances.

# F I G. 1

F I G. 2

FIG. 3

F I G . 4

F I G.  5

bodily impedance
measuring means  ~11

~12

data inputting
means

~13
fat rate
determining means

~14
memory
means

~15
display
means

FIG. 6

H

HL                                    HR

H1        ,H2              H2        H1

# F I G. 7

F I G. 8

BODILY FAT RATE (%)

number of exercises ⟶

F I G. 9

BODILY WEIGHT (Kg)

bodily fat (Kg)          non-fat texture weight (Kg)

number of exercises ⟶

# INTERNATIONAL SEARCH REPORT

| International application No. |
|---|
| PCT/JP99/02247 |

| A. CLASSIFICATION OF SUBJECT MATTER |
|---|
| Int.Cl⁶ A63B22/06, 23/04, 71/06, A61B5/05 |

According to International Patent Classification (IPC) or to both national classification and IPC

| B. FIELDS SEARCHED |
|---|
| Minimum documentation searched (classification system followed by classification symbols) |
| Int.Cl⁶ A63B22/06, 23/04, 71/06, A61B5/05 |

Documentation searched other than minimum documentation to the extent that such documents are included in the fields searched
Jitsuyo Shinan Koho 1922-1996 Toroku Jitsuyo Shinan Koho 1994-1999
Kokai Jitsuyo Shinan Koho 1971-1999 Jitsuyo Shinan Toroku Koho 1996-1999

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| Y | JP, 6-36839, Y2 (YAMAN Ltd.), 28 September, 1994 (28. 09. 94), Full text ; Figs. 1 to 5 (Family: none) | 1 |
| Y | JP, 8-126632, A (Omron Corp.), 21 May, 1996 (21. 05. 96), Full text ; Figs. 1 to 12 (Family: none) | 1 |
| Y | JP, 8-280840, A (Matsushita Electric Works,Ltd.), 29 October, 1996 (29. 10. 96), Full text ; Figs. 1 to 9 (Family: none) | 1 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| 22 July, 1999 (22. 07. 99) | 3 August, 1999 (03. 08. 99) |

| Name and mailing address of the ISA/ | Authorized officer |
|---|---|
| Japanese Patent Office | |
| Facsimile No. | Telephone No. |

Form PCT/ISA/210 (second sheet) (July 1992)